(19)

Europäisches
Patentamt
European
Patent Office
Office européen
des brevets

(11) **EP 2 289 557 A1**

(12) **EUROPEAN PATENT APPLICATION**

(43) Date of publication:
**02.03.2011 Bulletin 2011/09**

(51) Int Cl.:
*A61K 47/40* (2006.01)     *A61K 9/00* (2006.01)

(21) Application number: **10177383.6**

(22) Date of filing: **02.07.2004**

(84) Designated Contracting States:
**AT BE BG CH CY CZ DE DK EE ES FI FR GB GR HU IE IT LI LU MC NL PL PT RO SE SI SK TR**

(30) Priority: **04.07.2003 GB 0315745**

(62) Document number(s) of the earlier application(s) in accordance with Art. 76 EPC:
**04740601.2 / 1 646 405**

(27) Previously filed application:
**02.07.2004 PCT/EP2004/007253**

(71) Applicants:
• **Novartis AG**
**4056 Basel (CH)**
Designated Contracting States:
**BE BG CH CY CZ DE DK EE ES FI FR GB GR HU IE IT LI LU MC NL PL PT RO SE SI SK TR**
• **Novartis Pharma GmbH**
**1230 Wien (AT)**
Designated Contracting States:
**AT**

(72) Inventors:
• **Szente, Lajos**
**H-1118, Budapest (HU)**
• **Szejtli, Jozsef**
**H-1028, Budapest (HU)**
• **Jicsinszky, Laszlo**
**H-1192, Budapest (HU)**
• **Kis, Georg, Ludwig**
**CH-8273, Triboltingen (CH)**
• **Schoch, Christian**
**CH-4132, Muttenz (CH)**

(74) Representative: **Hutchison, John Robert**
**Novartis Pharma AG**
**Patent Department**
**4002 Basel (CH)**

Remarks:
This application was filed on 17-09-2010 as a divisional application to the application mentioned under INID code 62.

(54) **Pharmaceutical compositions comprising peranhydrocyclodextrine**

(57) The present invention relates to a pharmaceutical composition comprising a peranhydrocyclodextrin, a drug and a carries, to the use of peranhydrocyclodextrin as a drug transport enhancer (e.g., permeation enhancer), and to the use of a peranhydrocyclodextrin in the preparation of a pharmaceutical composition as a synergistic adjunctive system.

EP 2 289 557 A1

**Description**

The present invention relates to a pharmaceutical composition comprising a peranhydrocyclodextrin ,a drug and a carrier, to the use of a peranhydrocyclodextrin as a drug transport enhancer (e.g. permeation enhancer), and to the use of a peranhydrocyclodextrin in the preparation of a pharmaceutical composition as a synergistic adjunctive system.

The synthesis of peranhydrocyclodextrins was described as from 1991 (Gadelle A. and Defaye J., Angew. Chem. Int. Ed. Engl., (1991), 30, 78-79; Ashton P. R., Ellwood P., Staton I. and Stoddart J. F. Angew. Chem. Int. ed. Engl., (1991) 30, 80-81) and the authors describe that these derivatives have interesting solubilities both in water and in organic solvents.

As used herein, peranhydrocyclodextrins refer to per(3,6-anhydro)cyclodextrins, wherein the cyclodextrin may be alpha, beta or gamma or a mixture thereof, and wherein each molecule contains at least five 3,6-anhydro-glucopyranose units. Representative examples of said peranhydrocyclodextrins are hexakis(3,6-anhydro)-$\alpha$-cyclodextrin, heptakis(3,6-anhydro)-$\beta$-cyclodextrin, octakis(3,6-anhydro)-$\gamma$-cyclodextrin.

Typically, the per(3,6-anhydro)cyclodextrins of the alpha, beta or gamma cydodextins of the present invention may contain very small amounts of one non anhydrated glucopyranose units and tiny amounts of two non anhydrated glucopyranose units.

The amount of per(3,6-anhydro)cyclodextrin typically ranges from 0.0001 - 80% by weight of total composition, preferably from 0.001 - 70% by weight, more preferably from 0.01 - 65% and also from 0.1 - 60% by weight.

Pharmaceutical compositions comprising a pharmaceutically effective drug, a peranhydrocyclodextrin and a carrier are not described in the art.

Accordingly, in a first aspect the present invention pertains to a pharmaceutical composition, comprising a pharmaceutically effective drug, a per(3,6-anhydro)cyclodextrin and a carrier. A preferred pharmaceutical composition is a pharmaceutical composition for topical administration. A more preferred is an ophthalmic composition.

The compositions of the present invention seem to have a high permeation facilitating efficacy as compared to the prior art compositions, such as for example hydroxypropyl-gamma-cyclodextrin.

Accordingly another object of this invention is the use of a peranhydrocyclodextrin as a permeation enhancer and/or drug transport enhancer, virtually through any mammal tissue. Accordingly, the peranhydrocyclodextrins are useful in the enhancement of the bioavailability of any pharmaceutically effective drug.

In an embodiment the invention pertains to the use of a per(3,6-anhydro)cyclodextrin in the enhancement of the bioavailability of a pharmaceutically effective drug.

It also pertains to the use of a per(3,6-anhydro)cyclodextrin in the manufacture of a medicament for the enhancement of the bioavailability of a pharmaceutically effective drug.

The invention also pertains to the use of a per(3,6-anhydro)cyclodextrin to enhance drug permeation through cell membrane, wherein said membrane is preferably an ocular membrane, said drug being preferably administered topically to said cell membrane.

It further pertains to the use of a per(3,6-anhydro)cyclodextrin in the manufacture of a topical pharmaceutical medicament for the treatment of a disease being treatable by topical treatment, wherein said medicament comprises a per(3,6-anhydro)cyclodextrin, a carrier and a drug.

The invention further pertains to a method of improving drug permeability through (mammalian) tissue (through cell membrane), which method comprises the steps of: Preparing a pharmaceutical composition which comprises a per(3,6-anhydro)cyclodextrin, an effective amount of a drug, and a carrier, by conventionally admixing the individual components; and Administering said pharmaceutical composition to said tissue.

While applicant does not wish to be bound to any theory, applicant currently considers the following aspects regarding cell membrane permeation and the enhancement thereof:

Eventually, the peranhydrocyclodextrins of the present invention may utilize cation binding cyclodextrins in order to alter normal physiological functions of membrane ion-channels and pumps, the cation-dependent energy sources resulting in an enhanced drug permeation across biological membranes.

It is currently believed in the art that all membrane transport processes require:

-    permeability of the substance through the lipid bilayer, and
-    availability of an energy source for transport

The latter factor appears to be related - among others, and as described in the state of the art - to $Ca^{++}$ Ions, since the $Ca^{++}$ ATP-ase enzyme shall be an integral membrane protein participating in most of the membrane transport processes.

The lipid bilayer of biological membranes shall be intrinsically inpermeable to ions and polar molecules. The permeability of such substances shall be conferred by two types of membrane proteins: the *pumps* and the *channels. Pumps* seem to use a source of free energy (mainly from ATP, active transport) to transport ions.

The *channels* seem to allow the flow of ions rapidly across membranes. (e.g. passive transport)

Anhydro-cyclodextrin derivatives of the present invention (in its function as permeation enhancers) may affect these membrane protein-related transport processes resulting in enhanced drug transport through biological membranes. The presence of anhydro cyclodextrins, moreover, may result in alteration of ion potentials in the outer surface of the membrane, thus changing the ion distribution in the extracellular and intracellular space. This could lead to the change of membrane physiological functions and hence may result in the observed enhanced transport.

As oral administration is the most common and convenient route of drug delivery, many strategies have been developed to tackle the various problems which are associated with poor oral bioavailability.

Although these individual strategies are successfully applied to some drugs, a low oral bioavailability is very often the result of multiple factors and therefore, still requires a clear improvement.

The present invention offers a solution to the above problem by the use of a per-anhydro-cyclodextrins in a pharmaceutical composition.

Pharmaceutically active drugs of the present invention are typically selected from:

- Anti-angiogenic drugs, such as VEGF-inhibitors, PKC-inhibitors, also antibodies, antibody fragments having selectivity to the VEGF- or the PKC-receptor, e.g. N-benzoylstaurosporine, 1-(3-Chloroanilino)-4-(4-pyridylmethyl) phthalazine,
- Anti-inflammatory drugs, such as steroids, e.g. dexamethasone, fluorometholone , hydrocortisone, prednisolone; or so-called non-steroidal anti-inflammatory drugs (NSAID) such as COX-inhibitors, e.g. diclofenac, valdecoxib, lumiracoxib, ketorolac, or indomethacin;
- Anti-allergic drugs, selected e.g. from FK506, 33-epi-chloro-33-desoxy-ascomycin, cromolyn, emadine, ketotifen, levocabastine, lodoxamide, norketotifen, olopatadine, and rizabene;
- Drugs to treat glaucoma (in particular intraocular pressure treatment), selected e.g. from latanoprost, 15‑keto-latanoprost, unoprostone isopropyl, betaxolol, clonidine, levobunolol and timolol;
- Anti-infective drugs, e.g. selected from ciprofloxacin, chloramphenicol, chlortetracycline, gentamycin, lomefloxacin, neomycin, ofloxacin, polymyxin B and tobramycin;
- Antifungal drugs, e.g. selected from amphotericin B, fluconazole and natamycin;
- Anti-viral drugs such as acyclovir, fomivirsen, ganciclovir, and trifluridine;
- Anesthetic drugs, e.g. selected from cocaine hydrochloride, lidocaine, oxybuprocaine and tetracaine hydrochloride;
- Myopia preventing/inhibiting drugs such as pirenzepine, atropine and the like;
- Miotics, e.g. selected from carbachol, pilocarpine and physostigmine;
- Carbonic anhydrase inhibitors, e.g. selected from acetazolamide and dorzolamide;
- Alpha blocking agents, e.g. selected from apraclonidine and brimonidine; and
- Antioxidants and/or vitamins, e.g. selected from ascorbic acid, $\alpha$-tocopherol, $\alpha$-tocopherol acetate, retinol, retinol acetate, and retinol palmitate.

Preferred drugs are selected from:

Anti-angiogenic drugs, anti-inflammatory drugs, anti-allergic drugs, drugs to treat glaucoma, and myopia preventing/inhibiting drugs.

Further preferred are anti-angiogenic drugs, anti-inflammatory drugs, anti-allergic drugs, drugs to treat glaucoma, anti-infective drugs, anti-fungal drugs, anti-viral drugs, anesthetic drugs, myopia preventing/inhibiting drugs, miotics, carbonic anhydrase inhibitors, alpha blocking agents antioxidants and/or vitamins.

As used herein, a pharmaceutically active drug is a drug in free form, in the form of a salt and/or as a mixture thereof.

The pharmaceutical compositions of this invention comprise, for example, enteral or parenteral administration forms from approximately 10 % to approximately 80 %, preferably from approximately 20 % to approximately 60 %, active ingredient (drug). Pharmaceutical compositions according to the invention for enteral or parenteral administration are, for example, in unit dose form, such as in the form of dragées, tablets, capsules or suppositories, and also ampoules. They are prepared in a manner known *per se*, for example by means of conventional mixing, granulating, confectioning, dissolving or lyophilising processes. For example, pharmaceutical compositions for oral administration can be obtained by combining the active ingredient with solid carriers, if desired granulating a resulting mixture, and processing the mixture or granules, if desired or necessary, after the addition of appropriate excipients, into tablets or dragée cores.

Suitable carriers are especially fillers, such as sugars, for example lactose, saccharose, mannitol or sorbitol, cellulose preparations and/or calcium phosphates, for example tricalcium phosphate or calcium hydrogen phosphate, and also binders, such as starch pastes using, for example, corn, wheat, rice or potato starch, gelatin, tragacanth, methylcellulose and/or polyvinylpyrrolidone, if desired disintegrators, such as the above-mentioned starches, also carboxymethyl starch, crosslinked polyvinylpyrrolidone, agar, alginic acid or a salt thereof, such as sodium alginate. Excipients are especially flow agents, flow conditioners and lubricants, for example silicic acid, talc, stearic acid or salts thereof, such as magnesium or calcium stearate, and/or polyethylene glycol. Dragée cores are provided with suitable, optionally enteric, coatings, there being used, *inter alia,* concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or dragée coatings, for example for identification purposes or to indicate different doses of active ingredient.

Other suitable carriers might be selected from water, mixtures of water and other preferred carriers, mixtures of water and water-miscible solvents, such as $C_1$- to $C_7$-alkanols, vegetable oils or mineral oils comprising from 0.5 to 5% by weight hydroxyethylcellulose, ethyl oleate, carboxymethyl-cellulose, polyvinyl-pyrrolidone and other non-toxic water-soluble polymers for pharmaceutical uses, such as, for example, cellulose derivatives, such as methylcellulose, alkali metal salts of carboxy-methylcellulose, hydroxymethylcellulose, hydroxyethylcellulose, methylhydroxypropyl-cellulose and hydroxypropylcellulose, acrylates or methacrylates, such as salts of polyacrylic acid or ethyl acrylate, polyacrylamides.

A preferred carrier may be water, mixtures of water and other preferred carriers, mixtures of water and water-miscible solvents.

Another suitable carrier may for example contain or consist of:

a bioerodible polymer being selected from the group consisting of polyhydroxy-acids, such as polylactic acid and polyglycolic acid; polyesters, polycarbonates, polyorthoesters, polyanhydrides, polycyanoacrylates natural polymers such as gelatin, alginates, pectins, tragacanth, karaya gum, xanthan gum, carrageenin, agar and acacia; celluloses, such as carboxymethylcellulose; hydroxyethylcellulose, hydroxypropylcellulose; methacrylate (co)polymers such as Eudragits, e.g. Eudragit RL PO, Eudragit RS PO; and/or
a bioadhesive polymer being selected from the group consisting of maltodextrin, celluloses,
such as carboxymethyl cellulose, hydroxyethyl cellulose; chitosans; hyaluronic acid;
polyacrylates e.g. carbopol; polycarbophils e.g. Noveon AA-1; polyvinylalcohol such as Mowiol 26-88; polyvinylpyrrolidone such as povidone K30, polymeric cyclodextrin (MW being above 10'000); synthetic products, such as polyvinyl methyl ether, polyethylene oxide or
mixtures of those polymers.

A preferred carrier is a polymer which contains a branched polyglycolide lactide ester of glucose, having a molecular weight of from 20,000 to 200,000 (star polymer I).

Another preferred carrier is a polymer which contains a polyol ester having a molecular weight of 20,000 to 200,000, said polyol ester consisting essentially of:

1) 0.06% to 10% by weight of a polyol residue selected from the group consisting of

i) a cyclic structure having 1 to 8 glucose units containing 4 to 30 hydroxyl groups and
ii) a linear structure of mannitol containing 3 to 6 hydroxyl groups; and

2) a polylactic or co-poly-lactic residue having a molecular weight of 5,000 to 85,000, said polyol ester having at least 3 of said hydroxyl groups in esterified form and having a star-shaped polymer structure, wherein said polyol residue forms a central part surrounded by said polylactic or co-poly-lactic acid residue, and said co-poly- lactic residue comprises glycolic acid (star polymer II).

Another preferred carrier is a polymer which comprises polylactic acid with an average molecular weight of from 2000 to about 7000.

A preferred carrier is selected from the group of star polymer I, star polymer II, polylactic acid and mixtures thereof.

The amount of a carrier used in a composition of the present invention is in the range of from 0.01 to approximately 99% by weight, preferably in the range of from 1 - 95% by weight, more preferably in the range of from 10 - 90% by weight, even more preferably in the range of from 15 - 85% by weight, and in the range of from 20 - 80% by weight.

Other orally administrable pharmaceutical compositions are hard gelatin capsules and also soft, sealed capsules made of gelatin and a plasticiser, such as glycerol or sorbitol. The hard gelatin capsules may comprise the active ingredient in the form of granules, for example in admixture with fillers, such as lactose, binders, such as starches, and/or glidants, such as talc or magnesium stearate, and if desired with stabilisers. In soft capsules the active ingredient is preferably dissolved or suspended in suitable liquids, such as fatty oils, paraffin oil or liquid polyethylene glycols, it likewise being possible for stabilisers to be added.

Suitable rectally administrable pharmaceutical compositions are, for example, suppositories that consist of a combination of the active ingredient with a suppository base material. Suitable suppository base materials are, for example, natural or synthetic triglycerides, paraffin hydrocarbons, polyethylene glycols or higher alkanols. Gelatin rectal capsules that comprise a combination of the active ingredient with a base material may also be used. Suitable base materials include, for example, liquid triglycerides, polyethylene glycols and paraffin hydrocarbons.

There are suitable for parenteral administration by infusion and/or injection especially aqueous solutions of an active ingredient in water-soluble form, for example in the form of a water-soluble salt, and also suspensions of the active ingredient, such as corresponding oily suspensions, there being used suitable lipophilic solvents or vehicles, such as fatty oils, for example sesame oil, or synthetic fatty acid esters, for example ethyl oleate or triglycerides, or aqueous suspensions that comprise viscosity-increasing substances, for example sodium carboxymethylcellulose, sorbitol and/or dextran, and optionally also stabilisers.

The compounds may also be administered topically in or around the eye, for example as eyedrops, ophthalmic suspensions or ointments, subconjunctival, peribulbar, retrobulbar or intravitreal injections, possibly with the use of slow-release devices, such as conjunctival inserts, microspheres or other periocular or intraocular depot devices.

Any other object of the present invention may be described only in any of the independent and / or dependant claims of the present application, and may therefore additionally form a basis for amending the present description.

Chemical Examples:

Example 1. Preparation of hexakis(3,6-anhydro)-$\alpha$-cyclodextrin potassium chloride pentadecahydrate.

Freshly dried $\alpha$-cyclodextrin (48.6 g, 0.050 mol) is dissolved in freshly opened dimethyl formamide (800 cm$^3$) at room temperature. N-bromosuccinimide (142.4 g, 0.80 mol), and triphenylphosphine (148.6 g, 0.57 mol) is added in one portion to the solution at room temperature. The color of the reaction mixture becomes orange like as the TPP is added. The reaction temperature increases up to about 100 C at the end of TPP addition. The reaction mixture is immersed onto a pre-heated (60-70 C) oil-bath and stirred for 3 hrs at 80-85 C. When the reaction is completed, the reaction mixture is poured onto icy water (3000 cm$^3$). Ultrasonicated for several minutes and allowed to stand for crystallization (overnight). The yellow solid is filtered, washed with water (3 times 500 cm$^3$, pH= 2-3, 3-4, 4-5), dried under reduced pressure (5-10 kPa at moderate temperature (40-45 C) in the presence of P$_2$O$_5$. The obtained solid (250.6 g) contains TPPO and brominated a-cyclodextrins. The product is used for the preparation of anhydro-$\alpha$-cyclo-dextrin without further purification.

Potassium hydroxide (112.2 g, 2.0 mol) is dissolved in 3:1 MeOH/H$_2$O (1500 cm$^3$) at room temperature and tetrabromo-$\alpha$-cyclodextrin (250.6 g) is added spoonwise without additional cooling. The color of the reaction mixture disappears, then becomes opaque or precipitation is formed, the re-dissolution of the solid is observed and the solution again becomes yellow, which turns to brown upon elongated heating. The reaction is practically complete as the addition is finished. After 60 min the reaction mixture is cooled, stirrer is removed and methanol is removed by evaporation. When the methanol is removed, 200 cm$^3$ water is added. As the reaction mixture is free from MeOH, cooled to room temperature and neutralized (pH: -5.5) by cc. HCl. Charcoal is added to the suspension (20 g), and stirred for 1 hr. Filtered and washed with water (3 times 40 cm$^3$), and water is removed by freeze-drying (25 C/0.06-0.1 Pa). The obtained solid (light brown solid 220 g), contains KCl and KBr. TLC has few information about the composition due to the high inorganic salt content. The obtained product (220 g) is refluxed with MeOH (550 cm$^3$) for 30 min, and allowed to stand for crystallization (overnight).

The precipitate is filtered off, washed with MeOH (2*100 cm$^3$) and MeOH is removed by evaporation (55-60 C/10-15 kPa then 95-100 C/0.05-0.1 kPa, dark brown solid foam 29.8 g). The solid obtained from the evaporation (59.6 g) is dissolved in water (1200 cm$^3$). The pH of the solution is adjusted (pH:8.7 => 5.5) with 1 N HCl (62 cm$^3$) and clarified with charcoal (15 g) stirred at 25 C (overnight), filtered, washed with water (3 times 100 cm$^3$) and water was removed (58 g, 40 C/1-2 kPa, then 95-100 C/1-2 kPa). The obtained oil is dissolved in water (60 cm$^3$) and allowed to crystallize. Filtration resulted in white, crystalline material (11.2 g) with KCl content < 11 %, and water content < 30.0 %, Mp: 246-247

C [dec], $[\alpha]_D^{25} = -73.5$ (air-dry substance), conductivity of 1 aq. solution: ~1100 $\mu$S/cm.

Example 2. Preparation of hexakis(3,6-anhydro)-α-cyclodextrin.

Hexakis(6-deoxy-6-bromo)-a-cyclodextrin (15.1 g, 0.01 mol) is suspended in 3:1 MeOH/H$_2$O (1500 cm$^3$) at room temperature and lithium hydroxide (10.0 g, 0.4 mol) was added and heated to reflux. When the reaction is completed (approx. 20 hrs), the reaction mixture is cooled by addition of dry-ice. The formed lithium carbonate is filtered off, and solvents are removed by evaporation. The obtained solid is treated with acetone (100 cm$^3$), then dissolved in methanol (100 cm$^3$) and ions are removed by addition of strong anion- (25 g) then cation-exchanger (40 g). The ion-free solution is clarified by charcoal (2 g) and removal of methanol resulted in almost white solid (3.0 g). Mp: 230-235 C, $[\alpha]_D^{25}$ = -82.5 (air-dry substance), conductivity of 1 aq. solution: ~25 μS/cm.

Example 3. Preparation of heptakis(3,6-anhydro)-β-cyclodextrin

Freshly dried β-cyclodextrin (22.7 g, 0.020 mol) is dissolved in freshly opened dimethyl formamide (400 cm$^3$) at room temperature. Iodine (81.2 g, 0.32 mol), and triphenylphosphine (78.2 g, 0.30 mol) is added in one portion to the solution at room temperature with additional external cooling. The reaction temperature heated up to about 80 C, and stirred for 4 hrs at 80-85 C. When the reaction is completed, the major part of DMF is removed by distillation, than poured onto methanol (2000 cm$^3$), and allowed to crystallize 1 week, room temperature). The yellow solid is filtered, washed with methanol (3*200 cm$^3$), dried under reduced pressure (5-10 kPa at moderate temperature (40-45 C) in the presence of P$_2$O$_5$. The obtained solid (34.3 g, 90 % theor. yield) does not contain TPPO.

Heptakis(6-deoxy-6-iodo)- β-cyclodextrin (10.8 g, 0.005 mol) is dissolved in dimethyl sulfoxide, and sodium hydroxide (7.0 g, 0.175 mol) is added at stirred for 10 hrs at 70 C. The reaction mixture is cooled to room temperature and treated with ion-exchangers (100 g of strong anion- and 100 g of strong cation-exchanger), ionexchangers are removed by filtration, washed with DMSO (3 times 100 cm$^3$), then DMSO is removed *in vacuo,* and the obtained waxy solid was treated with acetone. Solid is filtered off and washed with acetone (pale yellow, 4.8 g). Mp: 230-235 C,

$[\alpha]_D^{25}$ = -85.5  -85.5 (air-dry substance), conductivity of 1 aq. solution: -20 μS/cm.

Example 4. Preparation of octakis(3,6-anhydro)-γ-cyclodextrin

Freshly dried γ-cyclodextrin (25.9 g, 0.020 mol) is dissolved in freshly opened dimethyl formamide (400 cm$^3$) at room temperature. N-bromosuccinimide (57.0 g, 0.32 mol), and triphenylphosphine (78.2 g, 0.30 mol) is added in one portion to the solution at room temperature. The color of the reaction mixture becomes orange like as the TPP is added. The reaction temperature increases up to about 60 C at the end of TPP addition. The reaction mixture is immersed onto a pre-heated (70-80 C) oil-bath and stirred for 4 hrs at 80-85 C. When the reaction is completed, the reaction mixture is poured onto icy water (3000 cm$^3$). Ultrasonicated for several minutes and allowed to stand for crystallization (overnight). The yellow solid is filtered, washed with water (3 times 200 cm$^3$, pH= 2-3, 3-4, 4-5), dried under reduced pressure (5-10 kPa at moderate temperature (40-45 C) in the presence of P$_2$O$_5$. The obtained solid (229 g) contains TPPO and brominated γ-cyclodextrins. The product is dissolved in methanol and the pH of the solution is adjusted to pH 9-10 with sodium methoxide. The pH-shift results in crystalline precipitation of the product. The crystalline material is removed by filtration (28.8 g, 80 % theor. yield). Octakis(6-deoxy-6-bromo)-γ-cyclodextrin (10.8 g, 0.006 mol) is dissolved in dimethyl sulfoxide, and lithium hydroxide (6.0 g, 0.24 mol) is added at stirred for 10 hrs at 70 C. The reaction mixture is cooled to room temperature and treated with ion-exchangers (100 g of strong anion- and 100 g of strong cation-exchanger), ion-exchangers are removed by filtration, washed with DMSO (3 times 100 cm$^3$), then DMSO is removed *in vacuo*, and the obtained waxy solid was treated with acetone. Solid is filtered off and washed with acetone (pale yellow, 4.3 g). Mp:

230-235 C, $[\alpha]_D^{25}$ = -92.5  -92.5 (air-dry substance), conductivity of 1 aq. solution: ~10 μS/cm.

Biological Examples:

Corneal permeation experiments with diclofenac (Voltaren) formulations
1) Diclofenac 0.1% without thiomersal (similar to marketed Voltaren Ophtha
formulation, SDU)

| Time (min) | Average permeated amount (microgram) | S.D. |
|---|---|---|
| 0 | 0.00 | 0.00 |
| 30 | 0.00 | 0.00 |
| 60 | 0.00 | 0.00 |
| 90 | 0.22 | 0.2 |
| 120 | 0.69 | 0.42 |
| 180 | 1.98 | 0.88 |

2) Diclofenac 0.1 % with 2% HP-gamma-CD and without BAC

| Time (min) | Average permeated amount (microgram) | S.D. |
|---|---|---|
| 0 | 0.00 | 0.00 |
| 30 | 0.00 | 0.00 |
| 60 | 0.25 | 0.24 |
| 90 | 1.22 | 0.53 |
| 120 | 2.25 | 0.71 |
| 180 | 6.43 | 1.64 |

3) Diclofenac 0.1% with 2% hexakis-(3,6-anhydro)-alpha-CD and without BAC

| Time (min) | Average permeated amount (microgram) | S.D. |
|---|---|---|
| 0 | 0.00 | 0.00 |
| 30 | 1.15 | 1.73 |
| 60 | 6.11 | 2.92 |
| 90 | 10.51 | 2.90 |
| 120 | 16.45 | 3.36 |
| 180 | 28.41 | 4.39 |

4) Diclofenac 0.1% with 2% heptakis-(3,6-anhydro)-beta-CD and without BAC

| Time (min) | Average permeated amount (microgram) | S.D. |
|---|---|---|
| 0 | 0.00 | 0.00 |
| 30 | 0.13 | 0.28 |
| 60 | 5.45 | 1.75 |
| 90 | 12.08 | 2.85 |
| 120 | 20.42 | 3.49 |
| 180 | 35.33 | 3.74 |

5) Diclofenac 0.1% with 2% octakis-(3,6-anhydro)-gamma-CD and without BAC

| Time (min) | Average permeated amount (microgram) | S.D. |
|---|---|---|
| 0 | 0.00 | 0.00 |
| 30 | 0.00 | 0.00 |
| 60 | 1.76 | 0.33 |
| 90 | 4.97 | 0.68 |
| 120 | 8.12 | 1.02 |
| 180 | 14.50 | 1.58 |

BAC = benzalkonium chloride

HP-gamma-CD = hydroxypropyl-γ-cyclodextrin

QA-β-CD: quaternary ammonium beta-cyclodextrin

In the above experiments [item 2) & item 3, 4 and 5)] the efficacy in drug permeation is directly comparable with respect to the prior art situation (HP-gamma-CD) and embodiments of this invention, namely hexakis-(3,6-anhydro)-alpha-CD, heptakis-(3,6-anhydro)-beta-CD and octakis-(3,6-anhydro)-gamma-CD.

**Claims**

1. A pharmaceutical composition comprising a per(3,6-anhydro)cyclodextrin, a pharmaceutically effective drug and a carrier.

2. Composition of claim 1, wherein said per(3,6-anhydro)cyclodextrin is selected from the group consisting of hexakis (3,6-anhydro)-α-cyclodextrin, heptakis(3,6-anhydro)-β-cyclodextrin, octakis(3,6-anhydro)-γ-cyclodextrin, and mixtures thereof.

3. Composition of claim 1, wherein said composition is adapted to topical administration.

4. Composition of claim 1, wherein the amount of said peranhydrocyclodextrin is in a range of from 0.01 - 80% by weight of total composition.

5. Composition of claim 1, wherein said composition is adapted to an administration in or around the eye.

6. Use of a per(3,6-anhydro)cyclodextrin in the enhancement of the bioavailability of a pharmaceutically effective drug.

7. Use of a per(3,6-anhydro)cyclodextrin in the manufacture of a medicament for the enhancement of the bioavailability of a pharmaceutically effective drug.

8. A method of improving drug permeability through a tissue, which method comprises the steps of:

   Conventionally admixing an effective amount of a per(3,6-anhydro)cyclodextrin, an effective amount of a drug, a carrier, and optionally one or more further ingredients selected from the group of buffers, tonicity enhancing agents, preservatives, solubilizers, stabilizers/solubilizers,
   and complexing agents; and
   administering said pharmaceutical composition comprising said per(3,6-anhydro)cyclodextrin to said tissue.

9. Method of claim 8, wherein said tissue is selected from mucus tissue and ocular tissue, such as corneal epithelial cells and conjunctival cells.

10. Method of enhancing the bioavailability of a pharmaceutically effective drug, which method comprises conventionally admixing an effective amount of a per(3,6-anhydro)cyclodextrin, an effective amount of a drug, and a carrier.

| | Europäisches Patentamt European Patent Office Office européen des brevets | **EUROPEAN SEARCH REPORT** | Application Number EP 10 17 7383 |
| --- | --- | --- | --- |

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| X | FR 2 814 748 A (COMMISSARIAT ENERGIE ATOMIQUE) 5 April 2002 (2002-04-05) <br> * abstract * <br> * page 1, line 27 - page 2, line 3 * <br> * page 5, line 25 - page 6, line 13 * <br> * page 7, lines 4-9 * <br> ----- | 1-4 | INV. <br> A61K47/40 <br> A61K9/00 |
| X | US 6 544 964 B1 (DEBOUZY JEAN-CLAUDE ET AL) 8 April 2003 (2003-04-08) <br> * column 2, formulae (I) , (II); column 4, lines 26-31 * <br> ----- | 1 | |
| A | EP 0 403 366 A (COMMISSARIAT ENERGIE ATOMIQUE ; DARCY RAPHAEL (IE); UNIV DUBLIN (IE)) 19 December 1990 (1990-12-19) <br> * the whole document * <br> ----- | 1-10 | |
| A | DEBOUZY J C ET AL: "COMPARATIVE CATION CHELATING PROPERTIES OF PER (3,6-ANHYDRO),- AND PER (3,6-ANHYDRO 2-OME) ALPHA-CYCLODEXTRINS", PROCEEDINGS OF THE INTERNATIONAL SYMPOSIUM ON CYCLODEXTRINS, SANTIAGO DE COMPOSTELA, SPAIN, MAY 31-JUNE 3, 1998,, vol. 9th, 1999, pages 105-108, XP001009589, <br> * figure 4 * <br> * page 108, Conclusions * <br> ----- | 1-10 | TECHNICAL FIELDS SEARCHED (IPC) <br><br> A61K |
| | -/-- | | |

| | | | |
| --- | --- | --- | --- |
| The present search report has been drawn up for all claims | | | |

| Place of search | Date of completion of the search | Examiner |
| --- | --- | --- |
| Munich | 22 December 2010 | Villa Riva, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 ................................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 17 7383

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | GADELLE A ET AL: "SELECTIVE HALOGENATION AT PRIMARY POSITIONS OF CYCLOMALTOOLIGOSACCHARIDES AND A SYNTHESIS OF PER-3,6-ANHYDRO CYCLOMALTOOLIGOSACCHARIDES", ANGEWANDTE CHEMIE. INTERNATIONAL EDITION, VERLAG CHEMIE. WEINHEIM, DE, vol. 30, no. 1, 1991, pages 78-80, XP000606849, ISSN: 0570-0833 * the whole document * ----- | 1-10 | |
| A,D | ASHTON P R ET AL: "SYNTHESIS AN I CHARACTERIZATION OF PER-3,6-ANHYDRO CYCLODEXTRINS", ANGEWANDTE CHEMIE. INTERNATIONAL EDITION, VERLAG CHEMIE. WEINHEIM, DE, vol. 30, no. 1, 1991, pages 80-81, XP000606848, ISSN: 0570-0833 * the whole document * ----- | 1-10 | TECHNICAL FIELDS SEARCHED (IPC) |

The present search report has been drawn up for all claims

| Place of search | Date of completion of the search | Examiner |
|---|---|---|
| Munich | 22 December 2010 | Villa Riva, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                        

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 10 17 7383

This annex lists the patent family members relating to the patent documents cited in the above-mentioned European search report.
The members are as contained in the European Patent Office EDP file on
The European Patent Office is in no way liable for these particulars which are merely given for the purpose of information.

22-12-2010

| Patent document cited in search report | | Publication date | Patent family member(s) | | Publication date |
|---|---|---|---|---|---|
| FR 2814748 | A | 05-04-2002 | NONE | | |
| US 6544964 | B1 | 08-04-2003 | AU | 752287 B2 | 12-09-2002 |
| | | | AU | 8218198 A | 30-12-1998 |
| | | | DE | 69802285 D1 | 06-12-2001 |
| | | | DE | 69802285 T2 | 18-07-2002 |
| | | | EP | 0991670 A1 | 12-04-2000 |
| | | | FR | 2764525 A1 | 18-12-1998 |
| | | | WO | 9856829 A1 | 17-12-1998 |
| | | | HU | 0002298 A2 | 28-11-2000 |
| | | | JP | 2002504167 T | 05-02-2002 |
| | | | ZA | 9805079 A | 12-01-1999 |
| EP 0403366 | A | 19-12-1990 | DE | 69017091 D1 | 30-03-1995 |
| | | | DE | 69017091 T2 | 28-09-1995 |
| | | | FR | 2648464 A1 | 21-12-1990 |
| | | | IE | 902157 A1 | 02-01-1991 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GADELLE A. ; DEFAYE J.** *Angew. Chem. Int. Ed. Engl.,* 1991, vol. 30, 78-79

- **ASHTON P. R. ; ELLWOOD P. ; STATON I. ; STODDART J. F.** *Angew. Chem. Int. ed. Engl.,* 1991, vol. 30, 80-81